# FASCICULE DE BREVET EUROPEEN

(11) **EP 0 473 472 B1**
(45) Date de publication et mention de la délivrance du brevet: **04.10.1995**
(21) Numéro de dépôt: 91402066.4
(22) Date de dépôt: 24.07.1991
(51) Int. Cl.: G01N 33/24

(54) **Dispositif pour faire des mesures thermodynamiques sur des échantillons de substances provenant notamment de zones pétrolifères**
Gerät zum Durchführen von thermodynamischen Messungen an Proben, die insbesondere aus Erdölformationen stammen
Device for making thermodynamic measurements on samples coming preferably from oil formations

(30) Priorité: 28.08.1990 FR 9010802
(43) Date de publication de la demande: 04.03.1992
(73) Titulaire: INSTITUT FRANCAIS DU PETROLE, 92506 Rueil-Malmaison Cédex (FR)
(72) Inventeur: Behar, Emmanuel, F-95000 Jouy le Moutier (FR); Moracchini, Gérard, F-95580 Andilly (FR); Sanchez, José, F-95270 Viarmes (FR)

(56) Documents cités:
- FR-A- 2 435 038
- FR-A- 2 593 921
- US-A- 3 611 799
- US-A- 4 149 805
- US-A- 4 539 837
- US-A- 4 602 498

## Description

La présente invention concerne un dispositif pour faire des mesures thermodynamiques sur des échantillons de substances provenant notamment de zones pétrolifères, et plus particulièrement un dispositif compact pouvant être facilement transporté jusque sur les sites de production et réaliser des mesures thermodynamiques complètes en déchargeant si néccessaire, les opérateurs de la conduite de nombreuses séquences opératoires.

Comme on le sait, il est d'usage de conduire une étude des propriétés thermodynamiques d'échantillons extraits de puits forés en vue de la mise en production de gisements souterrains d'effluents tels que des effluents pétroliers. Par une bonne connaissance de ces propriétés thermodynamiques, il est possible d'évaluer les réserves enfouies, d'optimiser l'installation de mise en production ou bien encore de spécifier les méthodes opératoires. Les propriétés thermodynamiques sont calculées en utilisant des modèles compositionnels dans lesquels on intègre des données obtenues par l'analyse des échantillons. Une méthode d'analyse du type dit "PVT" permet par exemple de mesurer le comportement d'échantillons dont on fait varier certains paramètres tels que la pression et la température par exemple depuis des valeurs reproduisant celles qui existent à leur profondeur d'enfouissement jusqu'à celles régnant en surface. Par des méthodes d'analyse de ce type, on arrive à mesurer des propriétés importantes telles que le "point bulle" indiquant l'apparition d'une phase gazeuse dans l'échantillon testé, le coefficient de compressibilité, la viscosité, la densité, le G.O.R (gas-oil ratio) etc, comme il est bien connu des spécialistes.

La plupart des dispositifs existants dans ce domaine, sont installés dans des laboratoires spécialisés. Les échantillons prélevés dans les puits et/ou des séparateurs d'essais, conditionnés dans des bouteilles pressurisées, doivent donc être centralisés vers ces installations d'analyse, ce qui est très pénalisant lorsque les puits sont répartis sur des zones étendues ou géographiquement très éloignées. En outre, ces dispositifs sont souvent conçus pour traiter à chaque fois des quantités d'échantillons relativement importantes en comparaison de celles que peuvent contenir les bouteilles de conditionnement couramment employées. De ce fait, on peut difficilement multiplier les cycles de mesure sur les échantillons transférés. Il faut noter que le traitement dans ces appareils, de quantités d'échantillons relativement importantes aux pressions et aux températures élevées que l'on rencontre dans les forages, exige des conditions de sécurité et de surveillance particulières.

On doit aussi noter que nombre d'appareils de laboratoire classiques utilisent du mercure pour mettre sous pression les cellules d'essai où les échantillons sont introduits à des fins de mesure. Cet agent de compression présente on le sait des inconvénients en raison de son poids, de sa toxicité élevée et de son coefficient de dilatation dont il faut tenir compte pour corriger certains résultats de mesure.

Des dispositifs adaptés à l'analyse thermodynamique d'échantillons sont décrits par exemple dans la demande de brevet français publiée FR-A-2 593 921 ou les brevets US No. 4 530 234, 4 660 413 ou 4 602 498.

Le dispositif selon l'invention permet de conduire directement sur le site d'extraction, des études thermodynamiques sur des échantillons de substances extraites du sous-sol, provenant notamment de zones pétrolifères. Il comporte une première chambre à volume variable délimitée dans un premier cylindre par un premier piston coulissant dans le premier cylindre sous l'action de moyens de déplacement, des moyens d'introduction dans la première chambre des échantillons à étudier, et des moyens de chauffage de ces échantillons.

Le dispositif est caractérisé en ce qu'il comporte une deuxième chambre à volume variable délimitée dans un deuxième cylindre par un deuxième piston coulissant sous l'action de moyens de déplacement pour recueillir au moins une partie de la substance introduite dans le premier cylindre, la deuxième chambre communiquant par un circuit pourvu d'une vanne avec la partie supérieure de la première chambre.

Suivant un mode de réalisation préféré, la partie supérieure de la première chambre est en forme de biseau, le circuit commandé par la première vanne débouchant à la pointe supérieure de la première chambre, laquelle est pourvue d'une paroi transparente permettant d'observer la partie supérieure de ladite première chambre.

La paroi transparente est par exemple celle d'un bloc transparent pourvu d'une cavité constituant ladite première chambre et le dispositif peut comporter un système optique dans ladite enceinte pour former hors de celle-ci, une image de la partie supérieure de la première chambre.

Les moyens de déplacement du premier et du deuxième piston comportant des moyens moteurs et des moyens de couplage débroyable pour déplacer le premier et le deuxième piston soit ensemble soit séparément.

Suivant un mode de réalisation préféré également, les deux chambres sont adaptées pour contenir des volumes réduits de substance, de l'ordre de quelques cm3 par exemple, et les moyens de déplacement du premier et du deuxième piston sont du type mécanique. De ce fait, aucun fluide auxiliaire tel que du mercure n'est nécessaire pour le fonctionnement des moyens d'entraînement.

Le dispositif comporte par exemple, entre la première et la deuxième chambre, un canal capillaire de dérivation associé à des moyens d'isolement et à un moyen de mesure de pression, pour effectuer des mesures de viscosité sur la substance contenue dans la première chambre.

Le dispositif peut comporter également un agitateur à ultrasons pour agiter la substance contenue dans la première chambre.

Suivant un mode de réalisation, le dispositif comporte une structure de support incluant un plateau supérieur pourvu d'une cavité cylindrique constituant la deuxième chambre, et un plateau inférieur pourvu d'une cavité cylindrique, un bloc transparent interposé entre les deux plateaux et traversé d'une cavité cylindrique constituant avec celle du plateau inférieur, ladite première chambre, les moyens de déplacement pour faire coulisser le premier et le deuxième piston comportant un premier étrier fixé au premier piston et un deuxième étrier fixé au deuxième piston, chacun d'eux étant solidaire en translation de deux tiges filetées pouvant coulisser dans des alésages aménagés respectivement dans les deux plateaux, et dans chacun de ces deux plateaux, des engrenages montés sur des arbres pour l'entraînement des tiges filetées, et des moyens de couplage amovibles pour connecter les arbres à des moyens moteurs.

Le dispositif comporte par exemple des vannes pour contrôler l'introduction d'échantillons de substance dans la première chambre et la communication de la deuxième chambre avec des appareils de mesure.

Suivant un mode de réalisation préféré, le dispositif comporte un ensemble de commande incluant un calculateur programmé pour conduire des opérations de mesure sur lesdits échantillons de substance introduits dans la première chambre.

Le dispositif selon l'invention est particulièrement adapté à être déplacé sur des lieux de prélèvement de substances à analyser.

Du fait du faible volume des deux chambres, on a obtenu une structure compacte et facilement transportable. Les quantités de substance mises en jeu dans chaque essai permettent tout à la fois de multiplier le nombre des analyses à partir d'une même bouteille de conditionnement, d'accélérer les étapes préparatoires et de faciliter le respect des contraintes de sécurité.

Avec sa première chambre au moins en partie transparente et terminée par une partie en biseau en haut duquel débouche le canal de communication avec la deuxième chambre et ses moyens optiques pour former l'image de cette chambre, on peut réaliser des séparations extrémement précises de la phase gazeuse de la substance testée.

L'emploi d'un dispositif à deux chambres dont les volumes peuvent être ajustés de façon précise permet des transferts dosés avec précision et évite l'utilisation par exemple d'un gazomètre à membrane qui fournit des résultats moins précis notamment quand les échantillons mesurés sont de faible volume.

L'emploi d'un ensemble de contrôle comportant un calculateur programmé pour conduire des séquences de mesures, permet de décharger l'opérateur d'une partie des tâches à réaliser durant les essais.

D'autres caractéristiques et avantages du dispositif selon l'invention apparaîtront mieux à la lecture de la description ci-après d'un mode de réalisation décrit à titre d'exemple non limitatif, en se référant aux dessins annexés où :
- la Fig.1 montre l'ensemble du dispositif avec son enceinte thermostatée pour la cellule d'essai, son boîtier de commande et l'ensemble de contrôle;
- la Fig.2 est une vue de face en coupe de la cellule d'essai;
- la fig.3 montre vue de face également et en coupe la partie centrale de la cellule d'essai avec les moyens d'entraînement des pistons;
- la Fig.4 montre une vue latérale de la cellule d'essai;
- la Fig.5 montre le schéma synoptique du boîtier adjacent à l'enceinte thermostatée;
- la Fig.6 montre le schéma synoptique de l'ensemble de contrôle du dispositif; et
- la Fig.7 est un schéma synoptique d'un ensemble d'instruments coopérant avec le dispositif de mesure.

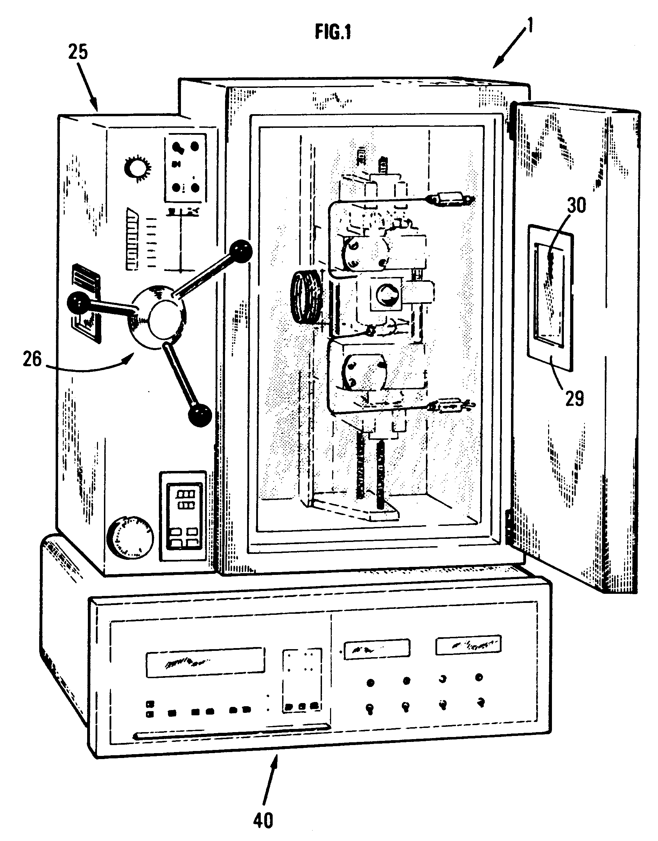

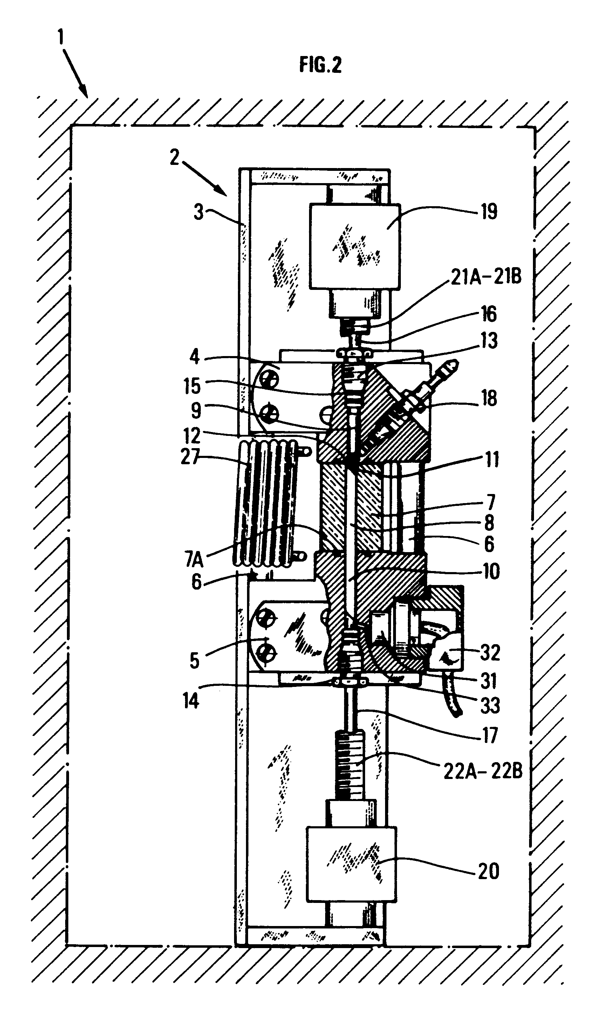

Le dispositif comporte (Fig.1) une enceinte thermostatée 1 à l'intérieur de laquelle est fixée rigidement une structure de support 2 (Fig.2-4). Cette structure comporte une plaque rigide 3 disposée verticalement à laquelle sont fixés l'un au-dessus de l'autre, deux plateaux 4, 5 réunis par des entretoises filetées 6. Dans l'espace entre les plateaux, est disposé un bloc cylindrique transparent 7, en saphir par exemple, pourvu d'un évidement axial 8 qui le traverse de part en part. Des cavités cylindriques et coaxiales 9 et 10, de même diamètre que la cavité 8, sont ménagées respectivement au travers du plateau supérieur 4 et du plateau inférieur 5. La cavité 10 dans le plateau inférieur 5, traverse celui-ci de part en part. Le plateau supérieur 4 est pourvu d'un prolongement tubulaire en forme de bec biseauté 11 de section égale à celle de l'évidement 8. Le bloc transparent 7 est positionné entre les deux plateaux avec engagement du bec 11 dans l'évidement 8, de manière que celui-ci soit dans l'alignement des deux cavités 9, 10 et interposition de moyens d'étanchéité 7A, pour établir une parfaite continuité bien étanche entre l'évidement 8 et la cavité 10 dans le plateau inférieur. Un canal fin 12 partant de l'arrière du bec à la pointe supérieure de l'évidement 8 dans le bloc transparent, fait communiquer celui-ci avec la cavité 9 ménagée dans le bloc supérieur 4. La pointe haute d'où part le canal 12 est visible de l'opérateur au travers du bloc transparent.

Du côté opposé au bloc transparent 7, les deux cavités 9 et 10 sont fermées par des embouts 13, 14 pourvus extérieurement de joints d'étanchéité 15 et traversés tous les deux par un alésage central de même section que les deux cavités 9, 10. Dans ces deux alésages coulissent de façon étanche deux tiges respectivement 16, 17 formant piston dans la deuxième chambre 9 et la première chambre 8, 10. L'extrémité du piston 16 est droite. Celle du piston 17 est biseautée et son biseau est orienté parallèlement à celui du bec 11.

On désigne par première chambre l'espace délimité par l'évidement 8, la cavité 10, le bec 11 et le piston biseauté 17. On désigne par deuxième chambre la partie de la cavité 9 délimitée par le piston 16.

La communication entre la première et la deuxième chambre par l'intermédiaire du canal 12 est contrôlée par une vanne 18.

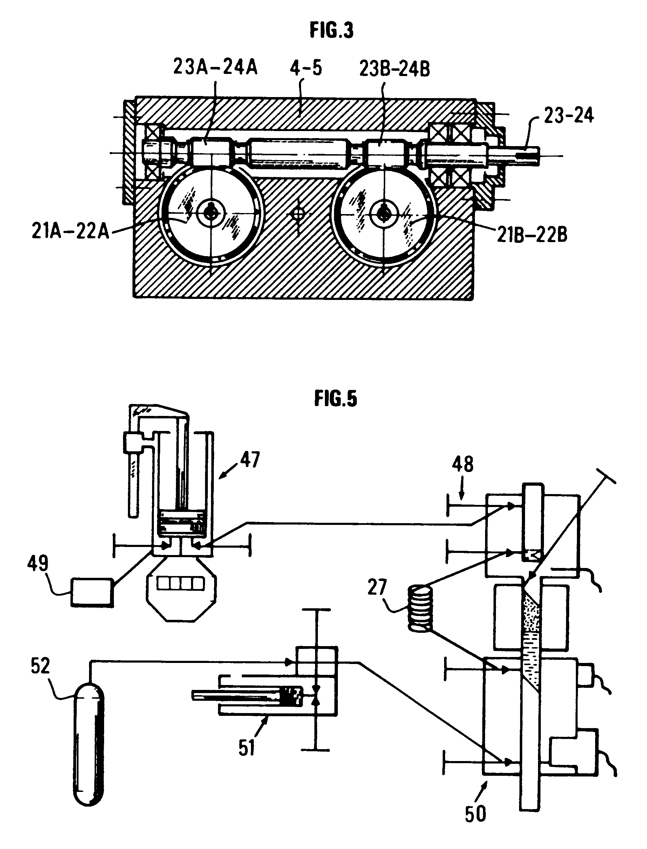

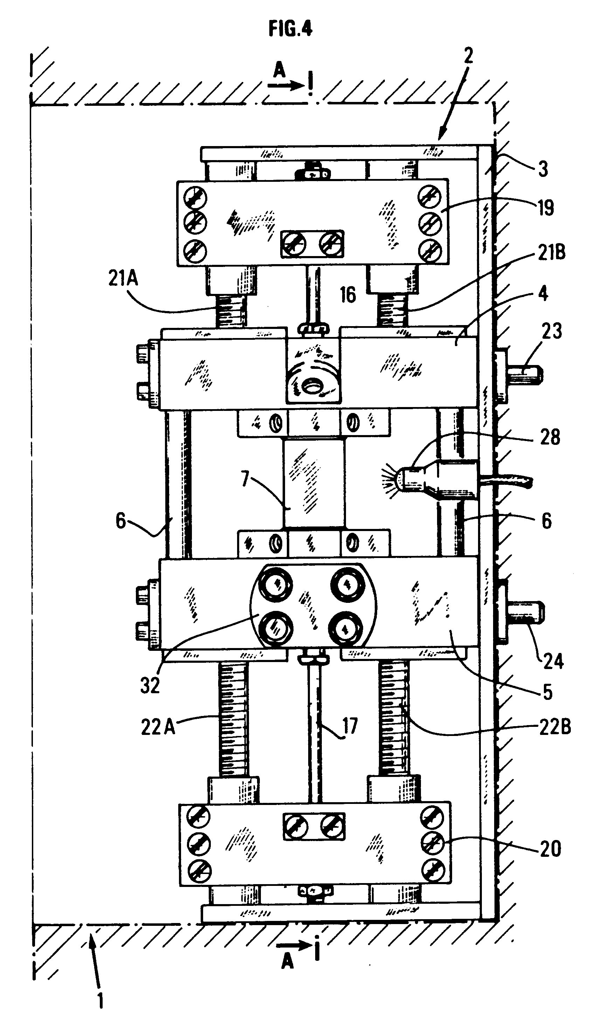

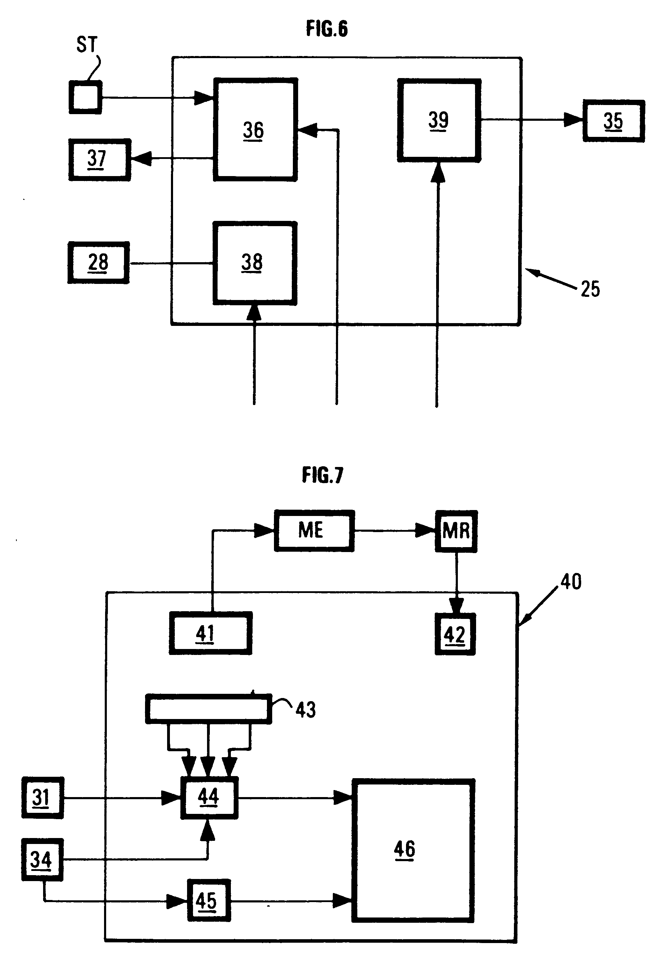

Les tiges 16, 17 formant piston, sont respectivement solidaires de deux étriers mobiles 19, 20 (Fig. 4) assujettis à se déplacer parallèlement à l'axe commun des deux chambres, relativement aux plateaux fixes 4, 5. A cet effet, deux tiges filetées respectivement 21A, 21B et 22A, 22B sont associées à chacun des étriers mobiles 19 et 20. Elles sont solidaires de leurs étriers respectifs en translation mais libres de tourner sur elles-mêmes. Le plateau 4 comporte deux alésages de guidage pour les tiges filetées 21A, 21B. De même le plateau 5 comporte deux alésages identiques pour les tiges filetées 22A, 22B. La rotation de chaque couple de tiges a pour effet de déplacer linéairement chaque étrier 19, 20 par rapport au plateau correspondant 4, 5. Ceci est obtenu dans chaque plateau au moyen d'un arbre d'entraînement 23 ou 24 (Fig. 3) disposé dans un plan perpendiculaire aux axes des tiges filetées. Chaque arbre est assujetti à un plateau 4, 5 mais pouvant tourner sur lui-même. Ils portent chacun deux engrenages (23A, 23B, 24A, 24B) venant s'engrener sur les filetages des tiges correspondantes 21, 22. Les sorties des deux arbres 23, 24 (Fig. 4) sont accouplées aux sorties d'un moto-réducteur MR associé à un moteur d'entraînement ME (Fig. 6), qui sont placés dans un boîtier adjacent 25 (Fig.1).

Les moyens d'accouplement des arbres aux sorties du moto-réducteur MR sont facilement amovibles et ainsi, l'ensemble de la cellule avec sa structure de support 2 peut être facilement extraite de l'enceinte 1. Un levier 26 en façade du boîtier 25 (Fig. 1), permet de sélectionner le mode d'entraînement manuel ou motorisé appliqué à chaque arbre 23 et 24 et aussi leur mode de fonctionnement soit séparément soit couplés, l'avancée du premier piston 17 dans la première chambre s'accompagnant par exemple du recul du deuxième piston 16 dans la deuxième chambre.

La section des chambres est suffisamment faible pour que l'on puisse faire des essais avec de petits volumes de substance, de l'ordre de quelques cm3 et les moyens moteurs sont adaptés à porter la pression dans ces chambres à plusieurs centaines de bars, par déplacement des tiges 16, 17 formant pistons. Les petits volumes mis en jeu permettent de minimiser les problèmes de sécurité d'une part et aussi la durée des étapes préalables de chauffage, d'agitation. Le dispositif selon l'invention convient particulièrement quand les quantités effectivement disponibles pour l'analyse sont faibles. C'est souvant le cas quand on fore des puits d'exploration par exemple. Le pétrole brut s'il y en a, est souvent réduit à l'état de traces dans les déblais remontés par la boue de forage. Le dispositif selon l'invention est néanmoins capable de faire des mesures thermodynamiques permettant à des ingénieurs de réservoir de tester les possibilités du puits. C'est encore le cas quand les forages atteignent des roches-mères. Le pétrole brut à analyser est extrait par pyrolyse des déblais de ces roches. Les quantités obtenues sont petites. Elles suffisent néanmoins au dispositif selon l'invention pour faire des mesures exploitables par les géo-chimistes qui cherchent à déterminer les sites les plus prometteurs.

L'échantillon de substance étant dans les conditions de température et de pression régnant dans la zone souterraine où il a été prélevé, on peut dans un premier temps abaisser sa pression par retrait du premier piston 17 suffisamment pour pouvoir mesurer par exemple sa compressibilité. En abaissant encore sa pression, on provoque sa vaporisation partielle. Par ouverture de la vanne 18, il est possible de transférer toute la phase gazeuse dans la deuxième chambre.

La communication entre les deux chambres peut se faire soit par l'intermédiaire de la vanne 18 comme on l'a vu, soit encore par l'intermédiaire d'un long capillaire enroulé 27 (Fig. 2) permettant de faire des mesures de viscosité du liquide introduit dans le dispositif, l'accès à ce capillaire étant commandé par une vanne non représentée.

Une source de lumière 28 est disposée à l'arrière de l'enceinte en face du bloc transparent 7 de manière à éclairer le haut du bec biseauté 11 d'où part le canal d'extraction 12. Une fenêtre transparente 29 est ménagée dans la porte avant de l'enceinte 1 pour laisser passer le pinceau lumineux issu de la source 28 et ayant traversé le bloc transparent 7. Un verre dépoli 30 est placé derrière la fenêtre 29 pour recueillir l'image de la pointe haute du bec 11.

Avec cette conformation particulière de la zone de prélèvement en haut de la première chambre, avec le bloc de saphir 7 et le système d'éclairement, on obtient une très grande précision dans toutes les opérations que l'on peut mener sur les fluides dans la première chambre. C'est vrai en particulier, comme on l'a vu, quand on doit prélever avec précision toute la phase gazeuse d'une substance diphasique sous pression dans cette première chambre pour l'introduire dans la deuxième chambre.

Un capteur de pression 31 est fixé dans une cavité 32 du plateau inférieur 5 et communique avec la première chambre par un canal 33. Le plateau supérieur est pourvu d'un logement pour une sonde de température 34.

Au plateau inférieur 5 peut être fixée une céramique piézo-électrique associée à un générateur d'ultrasons 35 (Fig. 6) pour agiter l'échantillon de substance introduit dans la première chambre.

Le boîtier 25 comporte (Fig. 6) un circuit 36 pour réguler la température. Ce circuit reçoit les données de température d'une sonde ST placée dans l'enceinte thermostatée et il agit sur les éléments de chauffage 37 de celle-ci, ce circuit étant adapté à limiter automatiquement la température maximale qui y règne, un organe de commande 38 de l'éclairage de la source de lumière 28 et un circuit 39 produisant le signal à appliquer au générateur d'ultrasons 35.

L'enceinte 1 et son boîtier adjacent 25 sont associés à un ensemble de contrôle 40 (Fig. 1, 7). Cet ensemble 40 comporte un circuit 41 de commande du moteur d'entraînement ME et un circuit de mesure par codage optique 42 d'un type connu, connecté au moto-réducteur MR, pour détecter les mouvements transmis aux arbres d'entraînement 23, 24 et les transformer en indication des variations de volume concomitantes de la première et de la deuxième chambre. L'ensemble 40 comporte aussi un circuit 43 de mémorisation des facteurs d'étalonnage du capteur de pression 31 en fonction de la température de consigne indiquée par la sonde de température 34 et un organe de sélection 44 des facteurs d'étalonnage à choisir en fonction de la température de consigne assignée, pour rendre la réponse du capteur 31 indépendante de celle-ci. La sonde de température 34 est connectée à un indicateur visuel 45. Différents témoins lumineux de contrôle non représentés permettent de visualiser des états et notamment des indications de fins de course. Une commande non représentée permet de sélectionner un mode de fonctionnement manuel sous le contrôle d'un opérateur ou un mode automatique sous le contrôle d'un calculateur 46 programmé pour conduire de façon automatique certaines séquences de mesure comme on le précisera plus loin.

Sur le schéma de la Fig. 5, on voit que le dispositif peut être associé à un gazomètre 47 que l'on relie à la deuxième chambre par une vanne 48. Avec ce gazomètre, on fait des mesures du volume du gaz prélevé dans la deuxième chambre après détente jusqu'à la pression atmosphérique. Le gazomètre est couplé par exemple avec un appareil de chromatographie 49. Par une vanne 50, la première chambre peut être couplée avec une micro-cellule de prélèvement d'un type connu 51 afin de mesurer la densité de la substance étudiée. Une petite quantité peut être prélevée sous pression dans la première chambre au moyen de cette cellule 51 et pesée.

Le transfert sous pression d'un échantillon dans la première chambre du dispositif peut être fait directement depuis une bouteille 52 de transport. On peut aussi l'introduire dans un stade intermédiaire dans la micro-cellule 51 avant de le transférer dans la première chambre.

Le calculateur 46 est adapté à réaliser certaines séquences d'acquisition et de conduite d'opérations.
- Il effectue par exemple un contrôle automatique de la pression lorsque à une certaine température de consigne, on transfère en le détendant le gaz de la deuxième chambre dans le gazomètre 47, et aussi une programmation de la température de consigne à respecter. Sur commande de l'opérateur il procède à une acquisition des données mesurées de pression, de volume et de température (P, V, T).
- Le calculateur 46 peut conduire aussi une séquence de décompression automatique avec stabilisations successives à plusieurs paliers de pression. Dans ce cas, il scrute la pression et attend sa stabilisation pour procéder à l'acquisition des données P, V, T avant une nouvelle décompression.
- Sur commande de l'opérateur il peut effectuer également toutes les mesures P, V, T nécessaires et les acquérir après validation, qu'il s'agisse de la pression à température de consigne, de la détermination des volumes de la première et de la deuxième chambre à une température de consigne, du volume de la phase gazeuse obtenue à la température ambiante et la pression atmosphérique.
- Le calculateur 46 comporte des moyens de mémorisation pour enregistrer les données acquises.
- Par programmation, on peut lui faire effectuer un certain nombre d'opérations classiques pour déterminer des paramètres thermodynamiques de l'échantillon étudié et/ou des paramètres d'étalonnage.

## Revendications

1. Dispositif pour faire des mesures thermodynamiques sur des échantillons de substances extraites du sous-sol et notamment de zones pétrolifères, comportant une enceinte (1) associée à des moyens (36, 37) pour y maintenir une température sensiblement constante, une première chambre (8, 10) à volume variable délimitée dans un premier cylindre dont l'axe est sensiblement vertical par un premier piston (17) coulissant dans le premier cylindre sous l'action de moyens de déplacement, des moyens d'introduction dans la chambre des échantillons à étudier, des moyens de chauffage de ces échantillons, une deuxième chambre (9) à volume variable délimitée dans un deuxième cylindre par un deuxième piston (16) coulissant sous l'action de moyens de déplacement pour recueillir au moins une partie de la substance introduite dans la première chambre, la deuxième chambre communiquant par un circuit (12) pourvu d'une première vanne (18) avec la partie supérieure de la première chambre, caractérisé en ce que les deux chambres sont adaptées pour contenir des petits volumes de substances, de l'ordre de quelques cm³ par exemple, la partie supérieure de la première chambre (8, 10) est en forme de biseau, le circuit (12) commandé par la première vanne (18) débouchant à la pointe supérieure de la première chambre laquelle est pourvue d'une paroi transparente (7) permettant d'observer ladite partie supérieure.

2. Dispositif selon la revendication 1, caractérisé en ce qu'il comporte un bloc transparent (7) pourvu d'une cavité cylindrique (8) et un système optique (28, 30) dans ladite enceinte pour former hors de celle-ci, une image de la partie supérieure en forme de biseau de la première chambre.

3. Dispositif selon la revendication 2, caractérisé en ce que le bloc transparent est réalisé en saphir.

4. Dispositif selon la revendication 1 ou 2, caractérisé en ce que les moyens de déplacement du premier et du deuxième piston comportent des moyens moteurs et des moyens de couplage débrayables pour déplacer le premier et le deuxième piston soit séparément soit ensemble et dans le même sens.

5. Dispositif selon la revendication 4, caractérisé en ce qu'il comporte un ensemble de contrôle (40) incluant des moyens de codage optique des déplacements des pistons, pour commander précisément les variations de volume des deux chambres.

6. Dispositif selon l'une des revendications précédentes, caractérisé en ce que les moyens d'entraînement du premier et du deuxième piston sont du type mécanique.

7. Dispositif selon l'une des revendications précédentes, caractérisé en ce qu'il comporte entre la première et la deuxième chambre, un canal capillaire de dérivation (27) associé à des moyens d'isolement et à un moyen de mesure de pression (31), pour effectuer des mesures de viscosité sur la substance contenue dans la première chambre.

8. Dispositif selon une des revendications précédentes, caractérisé en ce qu'il comporte un agitateur à ultrasons (35) pour agiter la substance contenue dans la première chambre.

9. Dispositif selon l'une des revendications 1 ou 4, caractérisé en ce qu'il comporte une structure de support (2, 3) incluant un plateau supérieur (4) pourvu d'une cavité cylindrique (9) constituant la deuxième chambre et un plateau inférieur (5) pourvu d'une cavité cylindrique, un bloc transparent interposé entre les deux plateaux et pourvu d'un évidement cylindrique (8) constituant ladite première chambre avec la cavité (10) du plateau inférieur (5), les moyens de déplacement pour faire coulisser le premier et le deuxième piston (17, 16) comportant un premier étrier (20) fixé au premier piston (17) et un deuxième étrier (19) fixé au deuxième piston (16), chacun d'eux étant solidaire en translation de deux tiges filetées (21A, 22A, 21B, 22B) pouvant coulisser dans des alésages ménagés respectivement dans les deux plateaux (4, 5), et dans chacun de ces deux plateaux, des engrenages (23A, 23B, 24A, 24B) montés sur des arbres (23, 24) pour l'entraînement des tiges filetées, et des moyens de couplage amovibles pour connecter ces arbres à des moyens moteurs (ME, MR).

10. Dispositif selon l'une des revendications précédentes, caractérisé en ce qu'il comporte des vannes (50) pour contrôler l'introduction d'échantillons de substance dans la première chambre et la communication de la deuxième chambre avec des appareils de mesure (48).

11. Dispositif selon l'une des revendications précédentes, caractérisé en ce qu'il comporte un ensemble de commande (40) incluant un calculateur programmé (46) pour conduire des opérations de mesure et de contrôle sur lesdits échantillons.

12. Dispositif selon la revendication 11, caractérisé en ce que l'ensemble de commande (40) comporte des moyens de mesure du volume respectifs de la première et de la deuxième chambre et des moyens (43, 44) pour rendre la réponse du moyen (31) de mesure de la pression, indépendante de la température de consigne imposée.

## Claims

1. A device for taking thermodynamic measurements on samples of substances extracted from the sub-soil and in particular from petroliferous zones, comprising a casing (1) connected to means (36, 37) to maintain a substantially constant temperature in the latter, a small chamber (8, 10) with a variable volume delineated in a first cylinder whose axis is substantially vertical by a first piston (17) sliding in the first cylinder as a result of the action of displacement means, means for introducing the analysis samples into the chamber, means for heating these samples, a second chamber (9) with a variable volume delineated in a second cylinder by a second piston (16) sliding under the action of displacement means to pick up at least a part of the substance introduced into the first chamber, the second chamber communicating with the upper part of the first chamber by means of a first valve (18), characterised in that the two chambers are designed to contain small volumes of substances in the order of a few cm³, for example, in that the upper part of the first chamber (8, 10) is bevel-shaped, the circuit (12) controlled by the first valve (28) opening into the upper part of the first chamber which is provided with a transparent wall (7) enabling this upper section to be observed.

2. A device as claimed in claim 1, characterised in that it comprises a transparent block (7) provided with a cylindrical cavity (8) and an optical system (28, 30) in this casing to form outside of it an image of the upper bevel-shaped part of the chamber.

3. A device as claimed in claim 2, characterised in that the transparent block is made from sapphire.

4. A device as claimed in claim 1 or 2, characterised in that the displacement means of the first and second pistons comprise motor means and detachable coupling means to displace the first and second pistons either separately or together and in the same direction.

5. A device as claimed in claim 4, characterised in that it comprises a control unit (40) incorporating means for optically coding the displacement of the pistons so as to control accurately the variations in the volume of the two chambers.

6. A device as claimed in one of the previous claims, characterised in that the drive means for the first and second pistons are of the mechanical type.

7. A device as claimed in one of the previous claims, characterised in that it comprises between the first and the second chamber a capillary by-pass channel (27) connected to isolating means and a pressure measuring means (31), to carry out viscosity measurements on the substance contained in the first chamber.

8. A device as claimed in one of the previous claims, characterised in that it comprises an ultra-sound agitator (35) to agitate the substance contained in the first chamber.

9. A device as claimed in one of claims 1 to 4, characterised in that it comprises a support structure (2, 3) incorporating an upper plate (4) provided with a cylindrical cavity (9) forming the second chamber and a lower plate (5) provided with a cylindrical chamber, a transparent block being inserted between the two plates and being provided with a cylindrical recess (8) forming the first chamber in conjunction with the cavity (10) of the lower plate (5), the displacement means for sliding the first and second pistons (16, 17) having a first stirrup clip (20) fixed to the first piston (17) and a second stirrup clip (19) fixed to the second piston (16), each of these being integral in translation with two threaded rods (21A, 22A, 21B, 22B) that may slide in bores arranged respectively in the two plates (4, 5) and in each of these two plates gears (23A, 23B, 24A, 24B) mounted on shafts (23, 24) to drive the threaded rods, and movable coupling means to connect these shafts to motor means (ME, MR).

10. A device as claimed in one of the previous claims, characterised in that it comprises valves (50) to control the introduction of substance samples into the first chamber and communication between the second chamber and measuring apparatus (48).

11. A device as claimed in one of the previous claims, characterised in that it comprises a control unit (40) including a computer programmed (46) to conduct measurement and control operations on these samples.

12. A device as claimed in claim 11, characterised in that the control unit (40) has means for measuring the respective volumes of the first and second chambers and means (43, 44) for making the response of the pressure measuring means (31) independent of the set temperature setting.

## Patentansprüche

1. Vorrichtung zum Durchführen von thermodynamischen Messungen an Proben von aus dem Untergrund und insbesondere aus ölführenden Zonen geförderten Substanzen, umfassend: einen Raum (1), der mit Einrichtungen (36, 37) zum dortigen Aufrechterhalten einer im wesentlichen konstanten Temperatur verbunden ist, eine erste Kammer (8, 10) mit variablem Volumen, die in einem ersten Zylinder, dessen Achse im wesentlichen vertikal ist, durch einen ersten Kolben (17) begrenzt ist, welcher in dem ersten Zylinder unter der Wirkung von Verschiebungseinrichtungen gleitet, Einrichtungen zur Einführung von zu untersuchenden Proben in die Kammer, Einrichtungen zur Erwärmung dieser Proben, eine zweite Kammer (9) mit variablem Volumen, die in einem zweiten Zylinder durch einen zweiten Kolben (16) begrenzt ist, welcher unter der Wirkung von Verschiebungseinrichtungen gleitet, um wenigstens einen Teil der in die erste Kammer eingeführten Substanz aufzunehmen, wobei die zweite Kammer über eine Leitung (12), die mit einem ersten Ventil (18) versehen ist, mit dem oberen Bereich der ersten Kammer kommuniziert, dadurch gekennzeichnet, daß die zwei Kammern geeignet sind, kleine Volumen der Substanzen, zum Beispiel in der Größenordnung von einigen cm³, einzuschließen, der obere Bereich der ersten Kammer (8, 10) in Keilform ausgebildet ist, wobei die durch das erste Ventil (18) gesteuerte Leitung (12) in den oberen Punkt der ersten Kammer einmündet, die mit einer durchsichtigen Wandung (7) versehen ist, welche gestattet, den oberen Bereich zu beobachten.

2. Vorrichtung nach Anspruch 1, dadurch gekennzeichnet, daß sie einen durchsichtigen Block (7) umfaßt, der mit einem zylindrischen Hohlraum (8) und einem optischen System (28, 30) in dem Raum versehen ist, um außerhalb dessen ein Bild des oberen Bereiches in Keilform der ersten Kammer zu bilden.

3. Vorrichtung nach Anspruch 2, dadurch gekennzeichnet, daß der durchsichtige Block aus Saphir realisiert ist.

4. Vorrichtung nach Anspruch 1 oder 2, dadurch gekennzeichnet, daß die Verschiebungseinrichtungen des ersten und des zweiten Kolbens Antriebseinrichtungen und ausschaltbare Einrichtungen zur Kupplung umfassen, um den ersten und den zweiten Kolben entweder getrennt oder gemeinsam und in der gleichen Richtung zu verschieben.

5. Vorrichtung nach Anspruch 4, dadurch gekennzeichnet, daß sie eine Einheit (40) zur Steuerung umfaßt, die Einrichtungen zur optischen Kodierung der Verschiebungen der Kolben beinhalten, um die Volumenänderungen der zwei Kammern genau zu steuern.

6. Vorrichtung nach einem der vorhergehenden Ansprüche, dadurch gekennzeichnet, daß die Einrichtungen zum Antrieb des ersten und des zweiten Kolbens von mechanischem Typ sind.

7. Vorrichtung nach einem der vorhergehenden Ansprüche, dadurch gekennezeichnet, daß sie zwischen der ersten und der zweiten Kammer einen kapillaren Umleitungskanal (27) umfaßt, der mit Einrichtungen zur Isolierung und mit einer Einrichtung (31) zur Druckmessung verbunden ist, um Messungen der Viskosität an der in der ersten Kammer enthaltenen Substanz durchzuführen.

8. Vorrichtung nach einem der vorhergehenden Ansprüche, dadurch gekennzeichnet, daß sie einen Ultraschallerzeuger (35) umfaßt, um die in der ersten Kammer enthaltene Substanz zu bewegen.

9. Vorrichtung nach einem der Ansprüche 1 oder 4, dadurch gekennzeichnet, daß sie eine Trägerstruktur (2, 3), die eine obere Platte (4), welche mit einem zylindrischen, die zweite Kammer bildenden Hohlraum (9) versehen ist, und eine untere Platte (5), welche mit einem zylindrischen Hohlraum versehen ist, beinhaltet, einen durchsichtigen Block, der zwischen den zwei Platten angeordnet und mit einer zylindrischen, die erste Kammer mit dem Hohlraum (10) der unteren Platte (5) bildenden Ausnehmung versehen ist, die Einrichtungen zur Verschiebung, welche einen ersten, an dem ersten Kolben (17) befestigten Abstandshalter (20) und einen zweiten, an dem zweiten Kolben (16) befestigten Abstandshalter (19) umfassen, um den ersten und den zweiten Kolben (17, 16) gleiten zu lassen, wobei jeder von ihnen zur Translationsbewegung aus einem Stück mit zwei Schraubenspindeln (21A, 22A, 21B, 22B) ist, die in Bohrungen, welche entsprechend in den zwei Platten (4, 5) angeordnet sind, und in Zahnrädern (23A, 23B, 24A, 24B), welche an Wellen (23, 24) zum Antrieb der Schraubenspindeln angebracht sind, in jeder dieser zwei Platten gleiten können, und lösbare Einrichtungen zur Kupplung, um diese Wellen mit den Antriebseinrichtungen (ME, MR) zu verbinden, umfaßt.

10. Vorrichtung nach einem der vorhergehenden Ansprüche, dadurch gekennzeichnet, daß sie Ventile (50) umfaßt, um die Einführung der Substanzproben in die erste Kammer und die Verbindung der zweiten Kammer mit den Geräten (48) zur Messung zu steuern.

11. Vorrichtung nach einem der vorhergehenden Ansprüche, dadurch gekennzeichnet, daß sie eine Einheit (40) zur Steuerung umfaßt, die einen programmierten Rechner (46) beinhaltet, um die Vorgänge zur Messung und zur Steuerung an den Proben durchzuführen.

12. Vorrichtung nach Anspruch 11, dadurch gekennzeichnet, daß die Einheit (40) zur Steuerung Einrichtungen zur Messung des entsprechenden Volumens der ersten und der zweiten Kammer und Einrichtungen (43, 44) umfaßt, um die Antwort der Einrichtung (31) zur Messung des Druckes unabhängig von dem vorgegebenen Einstelltemperaturwert zu liefern.
